# EUROPÄISCHE PATENTSCHRIFT

(11) **EP 1 491 166 B1**
(45) Veröffentlichungstag und Bekanntmachung des Hinweises auf die Patenterteilung: **02.03.2005**
(21) Anmeldenummer: 03020895.3
(22) Anmeldetag: 15.09.2003
(51) Int. Cl.: A61F 2/46

(54) **Einstellvorrichtung**
Adjusting device
Dispositif d'ajustage

(43) Veröffentlichungstag der Anmeldung: 29.12.2004
(73) Patentinhaber: Zimmer GmbH, 8404 Winterthur (CH)
(72) Erfinder: Overes, Thomas, 8404 Winterthur (CH)
(74) Vertreter: Manitz, Finsterwald & Partner GbR

(56) Entgegenhaltungen:
- EP-A- 0 979 636
- WO-A-02/17826
- US-A- 5 250 050
- US-A- 5 688 280

## Beschreibung

Die Erfindung betrifft eine Einstellvorrichtung zur Auswahl passender Implantatgrößen bei Knieoperationen mit einer an der Tibia abstützbaren unteren Arbeitseinheit und einer am Femur abstützbaren oberen Arbeitseinheit, die relativ zueinander höhenverstellbar sind und mit denen durch Aufspreizen des Knies der Abstand zischen der Tibia und dem Femur einstellbar ist.

Derartige Einstellvorrichtungen sind grundsätzlich bekannt, beispielsweise aus der EP 0 979 636 A2. Als nachteilig bei den bekannten Vorrichtungen wird zuweilen deren umständliche Handhabung empfunden.

Aufgabe der Erfindung ist es, eine Einstellvorrichtung der eingangs genannten Art zu schaffen, die möglichst einfach handhabbar ist und gleichzeitig eine sichere und zuverlässige Auswahl der passenden Implantatgröße während der Operation gestattet.

Die Lösung dieser Aufgabe erfolgt durch die Merkmale des Anspruchs 1 und insbesondere dadurch, dass die obere Arbeitseinheit einen relativ zur unteren Arbeitseinheit höhenverstellbaren Träger und wenigstens zwei verschiedene Funktionseinheiten umfasst, die mit dem Träger lösbar koppelbar sind und von denen die eine als zum Aufspreizen des Knies bei in Extension befindlichem Femur dienende Extensionseinheit und die andere als zum Aufspreizen des Knies sowie zum Auswählen einer passenden Implantatgröße bei in Flexion befindlichem Femur dienende Flexionseinheit ausgebildet ist, wobei die Flexionseinheit einen mit dem Träger koppelbaren Basisabschnitt und ein am Femur aufhängbares zentrales Führungsstück umfasst, das wahlweise in einen relativ zum Basisabschnitt in der Höhe frei bewegbaren oder festgesetzten Zustand bringbar ist, und wobei bei festgesetztem Führungsstück das Knie durch Verändern des Abstandes zwischen der unteren Arbeitseinheit und dem Träger aufspreizbar und bei frei bewegbarem Führungsstück der Basisabschnitt relativ zu dem am Femur aufhängbaren Führungsstück in der Höhe verstellbar ist.

Ein Vorteil der erfindungsgemäßen Einstellvorrichtung besteht darin, dass sie aufgrund der verschiedenen mit dem Träger koppelbaren Funktionseinheiten sowohl bei in Extension als auch in Flexion befindlichem Knie eingesetzt werden kann. Insbesondere kann der Operateur zunächst in Extension das Knie aufspreizen und in Abhängigkeit von der für ihn spürbaren Bänderspannung einen dem natürlichen Knie entsprechenden Abstand zwischen Tibia und Femur bestimmen. Dieser Abstandswert kann anschließend in Flexion als Bezugsgröße für die Auswahl der passenden Implantatgröße herangezogen werden. Hierzu braucht lediglich die Funktionseinheit ausgetauscht, d.h. anstelle der Extensionseinheit nunmehr die Flexionseinheit mit dem Träger gekoppelt zu werden. Die untere Arbeitseinheit der erfindungsgemäßen Einstellvorrichtung kann dabei im an der Tibia abgestützten Zustand verbleiben.

Ein weiterer Vorteil der erfindungsgemäßen Einstellvorrichtung ist das zentrale Führungsstück, das während der Operation am in Flexion befindlichen Femur aufgehängt und somit bezüglich der Aufspreizrichtung fest mit dem Femur verbunden ist. Erfindungsgemäß kann das zentrale Führungsstück bezüglich der Aufspreizrichtung, d.h. in der Höhe, wahlweise entweder am Basisabschnitt festgesetzt werden oder relativ zum Basisabschnitt frei bewegbar sein, d.h. freigegeben werden. Hierdurch ist es dem Operateur möglich, bei am Basisabschnitt festgesetztem Führungsstück die obere Arbeitseinheit als Ganzes anzuheben und somit das Knie aufzuspreizen. Bei festgesetztem zentralen Führungsstück ist folglich bezüglich der Aufspreizrichtung eine feste Relativlage zwischen Femur und Basisabschnitt gegeben, so dass bei festgesetztem Führungsstück der gewünschte Abstand zwischen Femur und Tibia eingestellt werden kann.

Bei relativ zum Basisabschnitt in der Höhe frei bewegbarem Führungsstück dagegen können der Basisabschnitt und das Femur, an dem das Führungsstück während der Operation aufgehängt ist, bezüglich der Aufspreizrichtung relativ zueinander bewegt werden. Folglich kann der Operateur bei freigegebenem Führungsstück den Basisabschnitt in die gewünschte Höhenposition relativ zum Femur bringen.

Die Erfindung bietet folglich mit einer einzigen Vorrichtung alle zur Auswahl der passenden Implantatgröße erforderlichen Einstellmöglichkeiten, und dies bei besonders einfacher Handhabung, denn zum einen kann die untere Arbeitseinheit während der gesamten Operation, d.h. sowohl während der Extensions- als auch während der Flexionsphase, ununterbrochen an der Tibia abgestützt bleiben, und zum anderen braucht lediglich zwischen dem festgesetzten und dem freigegebenen Zustand des zentralen Führungsstücks gewechselt zu werden, um wahlweise den Femur in die richtige Höhenposition bezüglich der Tibia oder des Basisabschnitts zu bringen und so beispielsweise durch iteratives Probieren die für das zu operierende Knie die am besten passende Implantatgröße schnell und einfach zu ermitteln.

Vorteilhafte Ausführungsformen der Erfindung sind in den Unteransprüchen, der Beschreibung sowie der Zeichnung angegeben.

Vorzugsweise ist erfindungsgemäß vorgesehen, dass das zentrale Führungsstück um eine in Aufspreizrichtung verlaufende Achse relativ zum Basisabschnitt verdrehbar ist. Hierdurch ist das Femur frei, seine natürliche Lage relativ zur Tibia einzunehmen.

Dabei ist es insbesondere bevorzugt, wenn das zentrale Führungsstück sowohl im frei bewegbaren als auch im festgesetzten Zustand relativ zum Basisabschnitt verdrehbar ist. Hierdurch ist sichergestellt, dass das Femur seine natürliche Lage relativ zur Tibia auch dann einnehmen kann, wenn es bei am Basisabschnitt festgesetztem Führungsstück aufgespreizt wird.

Das zentrale Führungsstück kann in einem an wenigstens zwei einander gegenüber liegenden Seiten offenen, in Aufspreizrichtung verlaufenden Führungskanal des Basisabschnitts in Längsrichtung des Führungskanal verschiebbar und um eine Längsachse des Führungskanals verdrehbar gelagert sein.

Das zentrale Führungsstück kann ferner wenigstens einen Durchgang aufweisen, durch den eine Aufhängeeinrichtung zum Aufhängen des Führungsstücks am in Flexion befindlichen Femur hindurchsteckbar ist. Bei der Aufhängeeinrichtung kann es sich insbesondere um einen Marknagel handeln.

Der Durchgang kann bezüglich einander diametral gegenüber liegender äußerer Funktionsbereiche, insbesondere Arretierungsstrukturen, schräg verlaufen. Hierdurch wird in vorteilhafter Weise eine Anpassung an den natürlichen Schrägverlauf des Femurs bezüglich der Tibia erreicht. Durch Wenden um 180°, d.h. gewissermaßen durch "Auf-den-Kopf-stellen" des Führungsstücks, kann zwischen der Operation eines linken Knies und der Operation eines rechten Knies gewechselt werden.

Ferner kann vorgesehen sein, dass das zentrale Führungsstück wenigstens zwei übereinander liegende Durchgänge aufweist. Auf diese Weise wird eine geringe Bauhöhe der Einstellvorrichtung ermöglicht, da durch gezielte Auswahl des Durchgangs für die Aufhängeeinrichtung insbesondere in Abhängigkeit von der Größe des Knies eine relativ kleine Führungslänge, d.h. ein relativ kleiner Verstellweg bei der Höhenverstellung des Basisabschnitts relativ zum Führungsstück realisiert werden kann.

Vorzugsweise weist das zentrale Führungsstück eine zylindrische Grundform auf.

Die Außenseite des zentralen Führungsstücks kann zumindest bereichsweise mit Arretierungsstrukturen versehen sein, mit denen eine Arretierungseinrichtung des Basisabschnitts zum wahlweisen Festsetzen oder Freigeben des Führungsstücks in und außer Eingriff bringbar ist. Bei den Arretierungsstrukturen kann es sich insbesondere um eine Verzahnung, Riffelung oder Nut-/ Rippenanordnung handeln.

Die Arretierungseinrichtung des Basisabschnitts kann eine in und außer Eingriff mit dem zentralen Führungsstück bewegbare Arretierungsbacke umfassen, die derart auf die Arretierungsstrukturen des Führungsstücks abgestimmt ist, dass im Eingriffszustand eine Höhenverstellung des Führungsstücks relativ zum Basisabschnitt unterbunden, ein Verdrehen des Führungsstücks dagegen möglich ist.

Ferner kann die Arretierungseinrichtung des Basisabschnitts einen mit einem Finger betätigbaren kombinierten Dreh-/Druckschalter umfassen.

Des Weiteren wird erfindungsgemäß vorgeschlagen, dass der Basisabschnitt ferner ein mit dem Träger koppelbares Unterteil und ein mit dem Unterteil gekoppeltes Oberteil umfasst, an dem das zentrale Führungsstück gelagert und das zusammen mit dem zentralen Führungsstück in mediolateraler Richtung relativ zum Unterteil verstellbar ist. Hierdurch kann das Femur nicht nur bezüglich der Aufspreizrichtung relativ zum Basisabschnitt verstellt werden, sondern es ist außerdem möglich, den Basisabschnitt bzw. dessen mit dem zentralen Führungsstück zusammenwirkendes Oberteil senkrecht zur Aufspreizrichtung, und zwar in mediolateraler Richtung, zu verstellen.

Das Oberteil kann als mit dem Unterteil lösbar zusammengesteckter Schlitten ausgebildet sein, der im Untereil in mediolateraler Richtung verschiebbar gelagert ist.

Ferner kann erfindungsgemäß vorgesehen sein, dass das Unterteil des Basisabschnitts mit einer Anzeigeeinrichtung versehen ist, an der das Femur durch Verstellen des Oberteils in mediolateraler Richtung ausrichtbar ist. Vorzugsweise ist die Anzeigeeinrichtung derart ausgebildet, das mittels der Anzeigeeinrichtung bezüglich des ausgerichteten Femurs passende und nicht passende Implantatgrößen voneinander unterscheidbar sind. Die Anzeigeeinrichtung kann in Form zweier seitlicher gestufter Flügelabschnitte vorgesehen sein, wobei die Stufen eine einfache visuelle Größenüberprüfung gestatten und jede Stufe einer vorhandenen und damit auswählbaren Implantatgröße entspricht.

Vorzugsweise umfasst die Flexionseinheit ferner einen relativ zum Basisabschnitt in der Höhe bewegbaren, zur Abstützung an der Vorderseite des in Flexion befindlichen Femurs ausgebildeten Femurtaster. Mittels eines derartigen Femurtasters kann bei einer bei freigegebenem Führungsstück möglichen Bewegung des Basisabschnitts relativ zum Femur die einer vorhandenen Implantatgröße entsprechende Relativlage zwischen Femur und Basisabschnitt eingestellt bzw. überprüft werden.

Der Basisabschnitt und/oder der Femurtaster können mit einer Anzeigeeinrichtung, insbesondere in Form einer Skala, versehen sein, an der ein von der Höhe des Basisabschnitts relativ zu dem Femurtaster abhängiges Maß für die Implantatgröße ablesbar ist.

Des Weiteren kann erfindungsgemäß vorgesehen sein, dass der Träger und/ oder die untere Arbeitseinheit mit einer Anzeigeeinrichtung, insbesondere in Form einer Skala, versehen ist, an der ein von der Höhe des Trägers relativ zu der unteren Arbeitseinheit abhängiges Maß für den Abstand zwischen der Tibia und dem Femur ablesbar ist.

Vorzugsweise ist erfindungsgemäß ferner vorgesehen, dass der Basisabschnitt, insbesondere dessen Unterteil, als Bohr- und/oder Schneidlehre ausgebildet ist. Hierdurch ist es möglich, sobald der Basisabschnitt bzw. dessen Unterteil korrekt bezüglich des in Flexion befindlichen Femurs positioniert ist, Bohrungen im Femur auszubilden, über die anschließend nach Abnahme der erfindungsgemäßen Einstellvorrichtung ein Schneidblock am Femur angebracht werden kann, mit dem das Femur entsprechend der mittels der erfindungsgemäßen Einstellvorrichtung ausgewählten passenden Implantatgröße bearbeitet werden kann.

Ferner kann vorgesehen sein, dass die untere Arbeitseinheit zur Höhenverstellung des Trägers einen Hebelmechanismus aufweist, der zusammen mit einem Handgriff der unteren Arbeitseinheit eine kombinierte Halteund Betätigungseinrichtung bildet, die derart ausgelegt ist, dass ein Benutzer mit einer Hand die Einstellvorrichtung als Ganzes am Handgriff halten und gleichzeitig den Hebelmechanismus zur Höhenverstellung des Trägers betätigen kann. Die Handhabung der erfindungsgemäßen Einstellvorrichtung wird hierdurch weiter vereinfacht.

Die Erfindung wird im Folgenden beispielhaft unter Bezugnahme auf die Zeichnung beschrieben. Es zeigen:
- Fig. 1a, 1b: verschiedene Ansichten einer Ausführungsform einer erfindungsgemäßen Einstellvorrichtung im zusammengesetzten Zustand, in dem eine Extensionseinheit mit dem höhenverstellbaren Träger gekoppelt ist,
- Fig. 2a, 2b: verschiedene Ansichten der Einstellvorrichtung von Fig. 1a, 1b mit an den Träger gekoppelter Flexionseinheit,
- Fig. 3a-3c: die untere Arbeitseinheit und den Träger der Einstellvorrichtung von Fig. 1a und 1b,
- Fig. 4a, 4b: verschiedene Ansichten der Flexionseinheit der Einstellvorrichtung in der Konfiguration von Fig. 2a, 2b,
- Fig. 5a-5d: verschiedene Ansichten des Oberteils und des zentralen Führungsstücks der Flexionseinheit von Fig. 4a, 4b im zusammengesetzten Zustand sowie eines in das Oberteil integrierten Dreh-/ Druckschalters,
- Fig. 6: das Oberteil von Fig. 5a, 5b ohne Führungsstück und ohne Dreh- / Druckschalter,
- Fig. 7a-7c: verschiedene Ansichten des zentralen Führungsstücks von Fig. 5a, 5b,
- Fig. 8: das Unterteil der Flexionseinheit von Fig. 4a, 4b,
- Fig. 9: den Femurtaster der Flexionseinheit von Fig. 4a, 4b, und
- Fig. 10: eine Seitenansicht von Fig. 4a zur Erläuterung der Funktionsweise des Femurtasters.

Der Ausgangspunkt für die Verwendung der erfindungsgemäßen Einstellvorrichtung ist eine Situation, in der bereits an der Tibia und am Femur jeweils ein Resektionsschnitt derart durchgeführt wurde, dass bei in Extension befindlichem Knie die beiden einander gegenüber liegenden Resektionsflächen parallel zueinander verlaufen. Die erfindungsgemäße Einstellvorrichtung dient dazu, diejenige Position zu finden, an welcher der hintere Femurschnitt durchzuführen ist, also der Femurschnitt, der zu einer ebenen Schnittfläche führt, die bei in Flexion befindlichem Knie parallel zur Resektionsfläche der Tibia verläuft.

Die "richtige" Position für diesen hinteren Femurschnitt zeichnet sich dadurch aus, dass unter Berücksichtigung der natürlichen Bänderspannung zum einen der Abstand zwischen den beiden Resektionsflächen in Extension möglichst gleich dem Abstand zwischen der Resektionsfläche der Tibia und der hinteren Femurschnittfläche in Flexion ist und zum anderen der hintere Femurschnitt derart gesetzt wird, dass die sich dadurch ergebenden anatomischen Verhältnisse am Femur möglichst genau einer vorgegebenen Implantatgröße entsprechen, da in der Praxis eine endliche Anzahl von Implantaten mit einer diskreten Größenverteilung zur Verfügung steht, unter denen die am besten passende Implantatgröße mittels der erfindungsgemäßen Einstellvorrichtung auszuwählen ist.

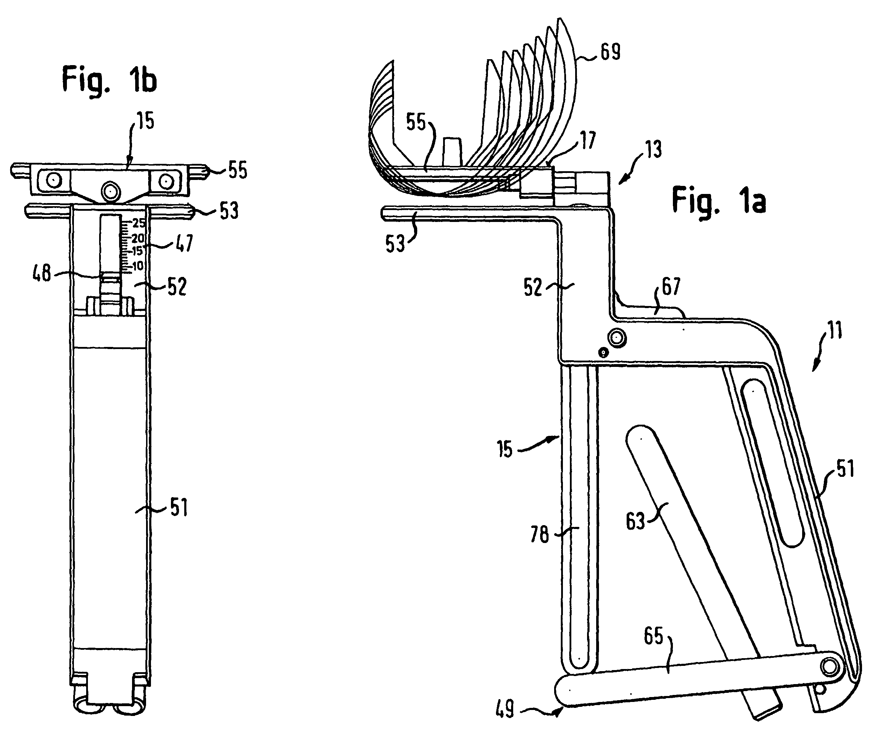

Die erfindungsgemäße Einstellvorrichtung umfasst gemäß Fig. 1a und 1b eine untere Arbeitseinheit 11 mit einem Handgriff 51 und einem Hebelmechanismus 49. Einstückig am Handgriff 51 ausgebildet ist eine untere Abstützplatte 53, über welche die untere Arbeitseinheit 11 und damit die gesamte Einstellvorrichtung an der Resektionsfläche der Tibia (nicht dargestellt) abgestützt werden kann.

Über den Hebelmechanismus 49 ist ein an anderer Stelle näher beschriebener, eine Tragstange 78 umfassender Träger 15 relativ zur unteren Arbeitseinheit 11 in der Höhe verstellbar. Hierzu wird ein erster Hebel 63 vom Operateur in Richtung des Handgriffs 51 gezogen, wodurch ein fest mit dem ersten Hebel 63 verbundener, an den Handgriff 51 angelenkter zweiter Hebel 65 die Tragstange 78 und damit den Träger 15 nach oben bewegt. Mittels einer ebenfalls an anderer Stelle näher erläuterten Feststelleinrichtung 67 kann der Träger 15 in einer jeweils eingestellten Höhe fixiert werden.

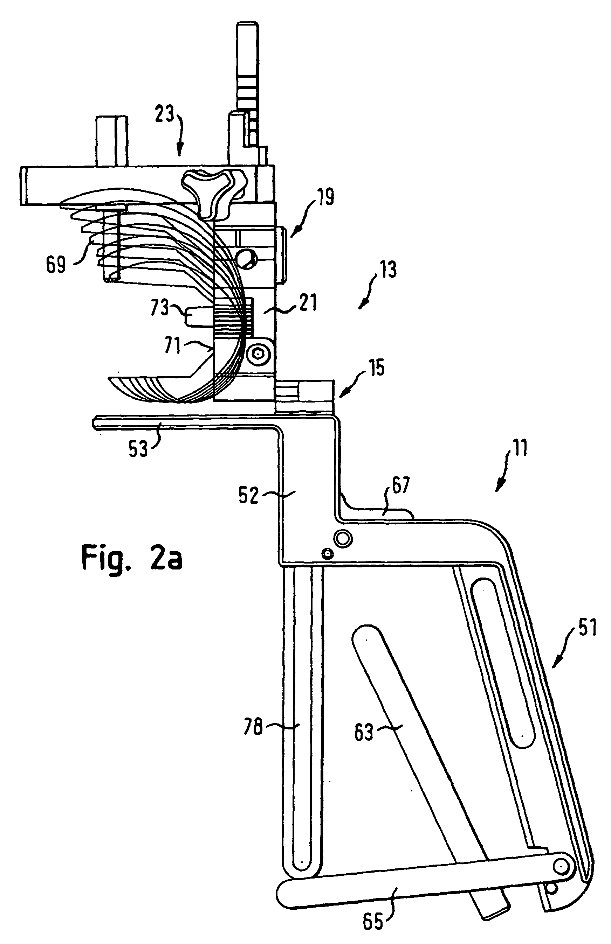

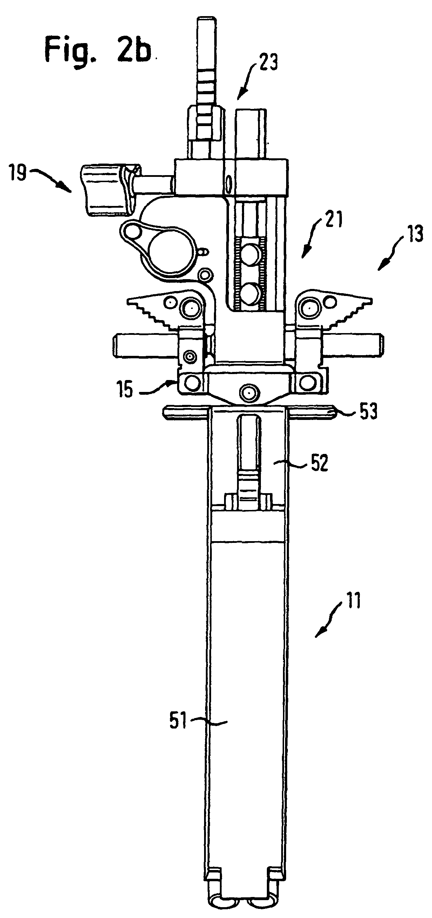

Der Träger 15 ist Bestandteil einer oberen Arbeitseinheit 13, die zusätzlich zu dem Träger 15 eine Extensionseinheit 17 und eine Flexionseinheit 19 (Fig. 2a und 2b) umfasst, die wahlweise durch Aufstecken mit dem Träger 15 gekoppelt werden können.

In Fig. 1a und 1b ist diese Funktionseinheit der oberen Arbeitseinheit 13 eine Extensionseinheit 17, die eine sich parallel zur Abstützplatte 53 der unteren Arbeitseinheit 11 erstreckende obere Abstützplatte 55 umfasst. Die obere Abstützplatte 55 liegt beim Aufspreizen des Knies an der Resektionsfläche des in Extension befindlichen Femurs an.

Folglich kann durch Betätigen des Hebelmechanismus 49 das in Extension befindliche Knie über die mit den Resektionsflächen zusammenwirkenden Abstützplatten 53, 55 aufgespreizt werden, wobei dies vom Operateur mit einer Hand bewerkstelligt werden kann, da er die gesamte Einstellvorrichtung mit einer Hand am Handgriff 51 halten und gleichzeitig den Hebelmechanismus 49 betätigen kann. Mit dieser Hand kann der Operateur außerdem die Feststelleinrichtung 67 betätigen, um den Träger 15 wahlweise freizugeben oder in der gewünschten Höhe zu arretieren.

In Fig. 1a sind zur Veranschaulichung mehrere Femurimplantate 69 dargestellt, unter denen das Implantat mit der am besten passenden Größe auszuwählen ist. Die Implantate 69 sind jeweils in derjenigen Position dargestellt, die sie relativ zu der erfindungsgemäßen Einstellvorrichtung einnehmen würden, wenn Einstellvorrichtung und Implantat 69 gleichzeitig bestimmungsgemäß am Knie positioniert wären. Fig. 1a lässt erkennen, dass der Abstand der Implantate 69 von der Resektionsfläche der Tibia, auf der die untere Abstützplatte 53 aufliegt, unabhängig von der Implantatgröße ist.

In der in Fig. 1a und 1b dargestellten Extensionskonfiguration dient die erfindungsgemäße Einstellvorrichtung dazu, das Knie durch Vergrößern des Abstands zwischen den beiden Resektionsflächen so weit aufzuspreizen, bis die natürliche Lage des Femurs relativ zur Tibia erreicht ist, wobei der Operateur diese natürliche Lage über die entsprechende, für ihn spürbare Bänderspannung ermitteln kann. Ein Maß für den "natürlichen" Abstand zwischen den beiden Resektionsflächen kann an einer Skala 47 abgelesen werden (Fig. 1b), die an dem sich in Aufspreizrichtung erstreckenden Übergangsabschnitt 52 zwischen Handgriff 51 und unterer Abstützplatte 53 ausgebildet ist. Der Träger 15 ist hierzu mit einem Abschnitt versehen, der als Zeiger 48 dient.

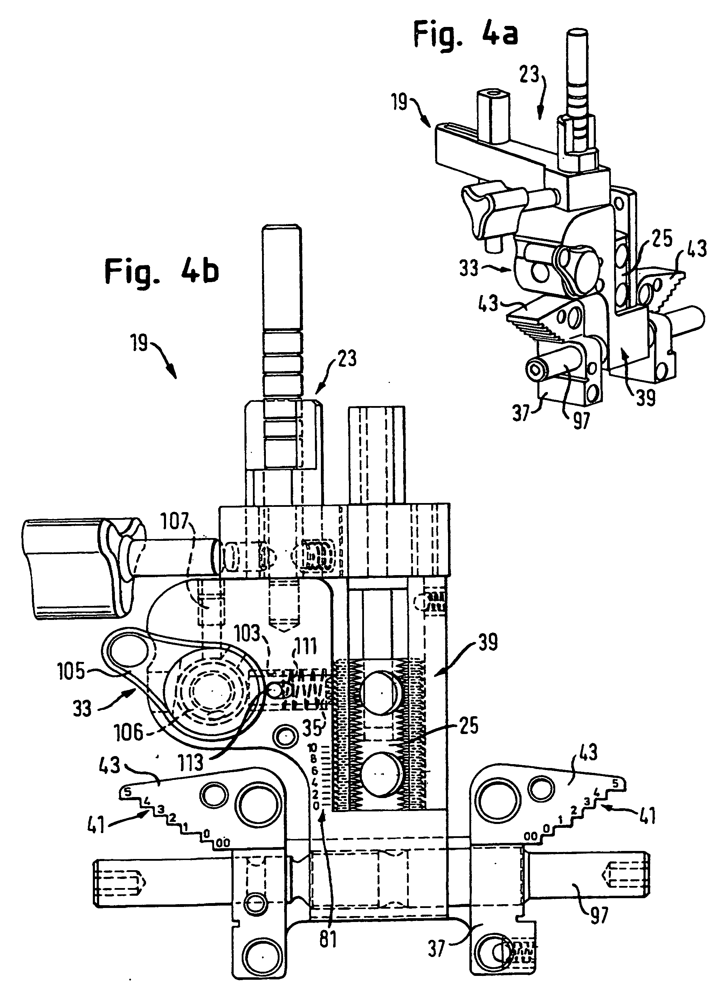

Die Fig. 2a und 2b zeigen die erfindungsgemäße Einstellvorrichtung in Flexionskonfiguration, in der auf den Träger 15 eine als Flexionseinheit 19 dienende Funktionseinheit aufgesteckt ist. Die Flexionseinheit 19 wird nachstehend in Verbindung mit den Fig. 4-8 näher beschrieben.

Fig. 2a zeigt entsprechend Fig. 1a die unterschiedlich großen Femurimplantate 69, hier jedoch in einer um 90° gegenüber Fig. 1a gedrehten Orientierung, da sich während des Einsatzes der Flexionseinheit 19 das Knie in Flexion befindet.

Aus Fig. 2a geht hervor, dass auch in Flexion der Abstand der Femurimplantate 69 von der Resektionsfläche der Tibia unabhängig von der Implantatgröße ist. Ebenfalls unabhängig von der Implantatgröße ist die Lage derjenigen Innenfläche 71 der Implantate 69, von der ein Verankerungsdorn 73 senkrecht absteht.

Die Flexionseinheit 19 der erfindungsgemäßen Einstellvorrichtung bietet alle Einstellmöglichkeiten, die erforderlich sind, um durch Variieren der Höhe sowie der mediolateralen Lage eines als Bohrlehre dienenden, nachstehend näher beschriebenen Bestandteils der Flexionseinheit 19 relativ zum Femur aus dem Satz von zur Verfügung stehenden Femurimplantaten 69 die am besten passende Implantatgröße auszuwählen, wobei die "am besten passende" Implantatgröße diejenige ist, die einen Bewegungsablauf ermöglicht, der dem natürlichen Bewegungsablauf des Knies möglichst nahe kommt.

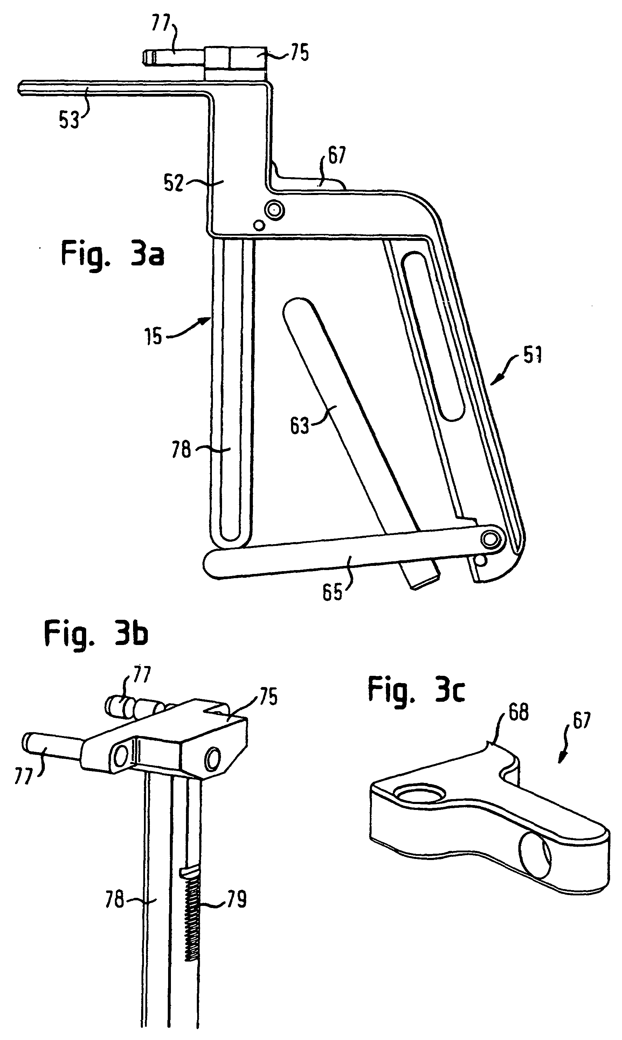

Fig. 3a zeigt den mit der unteren Arbeitseinheit 11 gekoppelten Träger 15 ohne aufgesteckte Funktionseinheit. Wie Fig. 3b zeigt, umfasst der Träger 15 ein Kopfteil 75 mit zwei eine Aufsteckgabel bildenden Tragstiften 77, auf welche die Funktionseinheiten - d.h. die Extensionseinheit 17 gemäß Fig. 1a und 1b und die Flexionseinheit 19 gemäß Fig. 2a und 2b - wahlweise aufsteckbar sind, sowie die Tragstange 78. Die Tragstange 78 ist bereichsweise mit einer Verzahnung oder Riffelung 79 versehen ist, in welche die in Fig. 3c dargestellte, gemäß Fig. 3a verschwenkbar an der unteren Arbeitseinheit 11 angebrachte Feststelleinrichtung 67 mit einem Arretierungszahn 68 verriegelnd eingreifen kann, um die Tragstange 78 und damit den Träger 15 zusammen mit der von ihm jeweils gehaltenen Funktionseinheit in der gewünschten Höhe relativ zu der unteren Arbeitseinheit 11 zu fixieren.

Gemäß Fig. 4a und 4b umfasst die Flexionseinheit 19 ein symmetrisch ausgebildetes Unterteil 37, ein quer - d.h. in mediolateraler Richtung - relativ zum Unterteil 37 verschiebbar am Unterteil 37 gelagertes Oberteil 39, das zusammen mit dem Unterteil 37 einen Basisabschnitt 21 (Fig. 2a, 2b) der Flexionseinheit 19 bildet, einen höhenverstellbar am Oberteil 39 angebrachten Femurtaster 23, ein zentrales Führungsstück 25 sowie eine in das Oberteil 39 integrierte Arretierungseinrichtung 33 zum wahlweisen Festsetzen und Freigeben des zentralen Führungsstücks 25 relativ zum Oberteil 39.

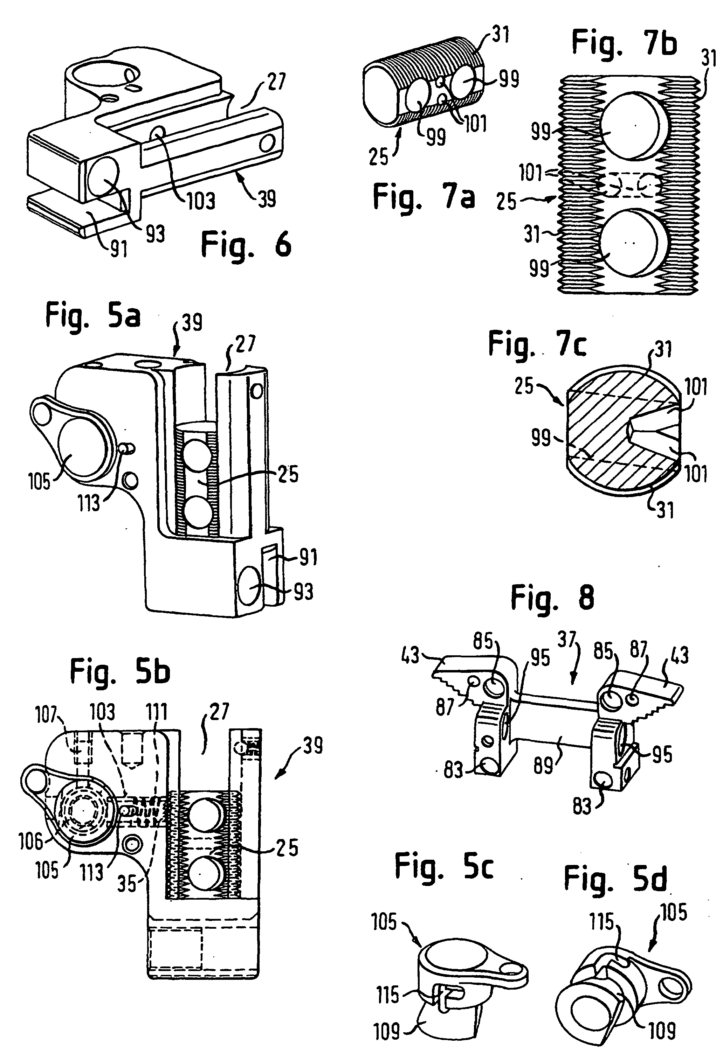

Nachstehend wird zur näheren Erläuterung der Flexionseinheit 19 auch auf die Fig. 5-8 Bezug genommen.

Das mit Trägerdurchgängen 83 zum Aufstecken auf die Stifte 77 des Trägers 15 (vgl. Fig. 3a, 3b) versehene Unterteil 37 ist mit zwei seitlichen gestuften Flügelabschnitten 43 versehen, deren Stufen eine Anzeigeeinrichtung 41 bilden, die dazu dient, das Femur in mediolateraler Richtung auszurichten und eine zur Auswahl in Erwägung gezogene Implantatgröße noch während der Operation daraufhin zu überprüfen, ob diese Implantatgröße im Hinblick auf die mediolateralen Abmessungen des Femurs bzw. der am Femur ausgebildeten Resektionsfläche verwendbar ist, d.h. ob das Implantat eine passende Breite aufweist.

Eine korrekte Lage des Femurs relativ zu dem Unterteil 37 auch in mediolateraler Richtung ist deshalb von Bedeutung, da das Unterteil 37 - das während der Operation zusammen mit dem Oberteil 39 flächig an der Resektionsfläche des in Flexion befindlichen Femurs anliegt - als Bohrlehre ausgebildet und hierzu mit Durchgängen 85, 87 versehen ist. Die großen Durchgänge 85 dienen als Bohrführungen für auch als "pegs" bezeichnete Stifte, die gleichzeitig als Bohrer und als Positionierstifte dienen und auf denen im Anschluss an den Einstellvorgang ein nicht dargestellter Schneidblock läuft. Der Zweck der erfindungsgemäßen Einstellvorrichtung besteht letztlich darin, die optimale Position für diese "pegs" und damit für den Schneidblock zu finden. Die kleinen, schräg verlaufenden Durchgänge 87 können dazu verwendet werden, so genannte "cross-pins" aufzunehmen, mit denen die Einstellvorrichtung am Knie fixiert wird.

Zwischen den beiden trillerpfeifenartig geformten Flügelabschnitten 43 des Unterteils 37 erstreckt sich ein flachquaderförmiger Steg 89, auf welchen das Oberteil (Fig. 5a, 6) von oben aufsteckbar und entlang welchem das Oberteil 39 quer - d.h. in mediolateraler Richtung - zum Unterteil 37 verschiebbar ist. Hierzu ist das Oberteil 39 mit einer zu dem Steg 89 passenden Schlitzausnehmung 91 versehen.

Das Oberteil 39 weist ferner einen sich parallel zur Schlitzausnehmung 91 erstreckenden Querdurchgang 93 mit kreisförmigem Querschnitt auf, der im zusammengesetzten Zustand gemäß Fig. 4a, 4b mit entsprechenden Querdurchgängen 95 des Unterteils 37 ausgerichtet ist. Über die Durchgänge 93, 95 werden das Oberteil 39 und das Unterteil 37 mittels einer Spindel 97 in Aufspreizrichtung fest zusammengehalten, so dass eine Höhenverstellung von Unterteil 37 und Oberteil 39 nur gemeinsam möglich ist. Durch Betätigen der Spindel 97 können das Oberteil 39 und das Unterteil 37 in Längsrichtung der Spindel 97, also senkrecht zur Aufspreizrichtung, relativ zueinander verschoben werden, um mittels der gestuften Flügelabschnitte 43 die vorstehend erwähnte Anpassung bzw. Messung in mediolateraler Richtung vorzunehmen.

Im Oberteil 39 ist des Weiteren ein in Aufspreizrichtung verlaufender Führungskanal 27 ausgebildet, der an zwei einander gegenüber liegenden Seiten offen ist und in den von oben das eine zylindrische Grundform aufweisende zentrale Führungsstück 25 einführbar ist. Der Führungskanal 27 und das Führungsstück 25 sind derart aufeinander abgestimmt, dass das Führungsstück 25 ohne wesentliches seitliches Spiel innerhalb des Führungskanals 27 in Aufspreizrichtung relativ zum Oberteil 39 verschiebbar und um eine parallel zur Aufspreizrichtung verlaufende, mit der Längsachse des Führungskanal 27 und des Führungsstücks 25 zusammenfallende Achse verdrehbar ist.

Zwei einander gegenüber liegende Außenseitenbereiche des Führungsstücks 25 sind jeweils mit einer von einer Nut-/Rippenanordnung gebildeten Verzahnung oder Riffelung 31 versehen, die Arretierungsstrukturen des Führungsstücks 25 bilden, in welche die nachstehend näher beschriebene Festsetzeinrichtung 33 des Oberteils 39 verriegelnd eingreifen kann, um das Führungsstück 25 in einer gewünschten Höhe bezüglich des Oberteils 39 festzusetzen.

Wie insbesondere die Fig. 7b und 7c zeigen (Fig. 7c ist ein Schnitt durch das Führungsstück 25 entlang der senkrecht zur Längsachse des Führungsstücks 25 verlaufenden Mittelebene), ist das Führungsstück 25 mit zwei in axialer Richtung beabstandeten Aufhängedurchgängen 99 versehen, die jeweils bezüglich der die Arretierungsstrukturen 33 bildenden Außenseitenbereiche nicht symmetrisch, sondern schräg durch das Führungsstück 25 verlaufen. Ferner ist das Führungsstück 25 zwischen den beiden Aufhängedurchgängen 99 in Höhe der zur Längsachse senkrechten Mittelebene mit zwei V-förmig verlaufenden Sackbohrungen 101 versehen, die an einer Außenseite des Führungsstücks 25 zwischen den strukturierten Bereichen 31 münden und vor Erreichen der in Längsrichtung verlaufenden Mittelebene des Führungsstücks 25 enden und ineinander übergehen. In diese Sackbohrungen 101 können nicht dargestellte Begrenzungsstifte eingesetzt, z.B. eingepresst, werden, die als Drehanschläge für das Führungsstück 25 dienen und die Verdrehbarkeit des Führungsstücks 25 im Führungskanal 27 derart begrenzen, dass stets der volle Querschnitt der Durchgänge 99 zum Einführen eines Marknagels zur Verfügung steht.

Während der Operation ist durch einen der Aufhängedurchgänge 99 des Führungsstücks 25 ein Marknagel (nicht dargestellt) hindurch geführt, der über die Resektionsfläche des Femurs, an der Oberteil 39 und Unterteil 37 anliegen, in das Femur eingesteckt ist, um das im Führungskanal 27 befindliche Führungsstück 25 und damit die Flexionseinheit 19 am Femur aufzuhängen. Um das Hindurchführen des Marknagels zu ermöglichen, ist der Führungskanal 27 an den beiden einander gegenüber liegenden Längsseiten offen.

Die Festsetzeinrichtung 33 des Oberteils 39 umfasst eine als Schiebestift ausgebildete Arretierungsbacke 35, die mit einer Feder 111 zusammenwirkt und in einem sich senkrecht zum Führungskanal 27 erstreckenden und in den Führungskanal 27 mündenden Querkanal 103 verschiebbar angeordnet ist. Auf ihrer mit dem Führungsstück 25 zusammenwirkenden Stirnseite ist die Arretierungsbacke 35 mit einer ebenfalls von einer Nut/Rippen-Anordnung gebildeten Riffelung oder Verzahnung versehen, die derart auf die Arretierungsstrukturen 31 des Führungsstücks 25 abgestimmt ist, dass die Strukturen derart ineinander greifen können, dass das Führungsstück 25 zwar an einer Bewegung relativ zum Oberteil 39 in Aufspreizrichtung gehindert, ein Verdrehen des Führungsstücks 25 relativ zum Oberteil 39 dagegen möglich ist.

Die Festsetzeinrichtung 33 umfasst ferner einen kombinierten Dreh/Druckschalter 105, der zum einen mit der im Querkanal 103 verschiebbaren Arretierungsbacke 35 und zum anderen mit einem Verriegelungsstift 107 zusammenwirkt. Die Fig. 5a und 5b (vgl. auch Fig. 4b) zeigen den Freigabezustand der Festsetzeinrichtung 33, in welchem sich die Arretierungsbacke 35 außer Eingriff mit dem Führungsstück 25 befindet. Der Schalter 105 befindet sich im eingedrückten Zustand, in dem er durch den Verriegelungsstift 107 gehalten wird, solange er nicht gegen den Uhrzeigersinn in Fig. 4b und Fig. 5a, 5b gedreht wird.

Wenn der Schalter 105 aus der Freigabestellung gedreht wird, gibt der Verriegelungsstift 107 den Schalter 105 frei, so dass er durch eine im Freigabezustand zusammengedrückte Feder 106 aus dem Oberteil 39 heraus nach außen gedrückt wird, wodurch das dem Schalter 105 zugewandte Ende der Arretierungsbacke 35 durch eine konische Steuerfläche 109 des Schalters 105 (Fig. 5c und 5d) in Eingriff mit dem Führungsstück 25 gedrückt wird. Diese Eingriffsbewegung erfolgt gegen die Rückstellkraft einer die Arretierungsbacke 35 umgebenden Feder 111, die von einem senkrecht abstehenden Stift 113 der Arretierungsbacke 35 gespannt wird, d.h. die den Schalter 105 heraus drückende Feder 106 kann die Rückstellkraft der Feder 11 überwinden.

Der eine kegelstumpfartige Form aufweisende, mit der konischen Steuerfläche 109 versehene Bereich des Schalters 105 ist nicht rotationssymmetrisch ausgeführt, sondern an einer Seite weggeschnitten.

Die Fig. 5c und 5d zeigen außerdem, dass der Schalter 105 eine L-förmige Steuernut 115 aufweist, in welche der Verriegelungsstift 107 eingreift. Über den sich in Umfangsrichtung erstreckenden Abschnitt der Steuernut 115 wird der Schalter 105 durch den Verriegelungsstift 107 in der eingedrückten, dem Freigabezustand gemäß Fig. 4b und 5a, 5b entsprechenden Stellung gehalten, während es der sich in radialer Richtung erstreckende Abschnitt der Steuernut 115 ermöglicht, dass der Schalter 105 nach entsprechender Drehung aufgrund der sich entspannenden Feder 106 heraus springen und über die konische Steuerfläche 109 die Arretierungsbacke 35 in Eingriff mit dem Führungsstück 25 schieben kann.

Wie insbesondere Fig. 5a zeigt, ist der Stift 113 der Arretierungsbacke 35 in einem Langloch des Oberteils 39 geführt, wodurch die korrekte Orientierung zwischen den Verzahnungen der Arretierungsbacke 35 und des Führungsstücks 25 sichergestellt ist.

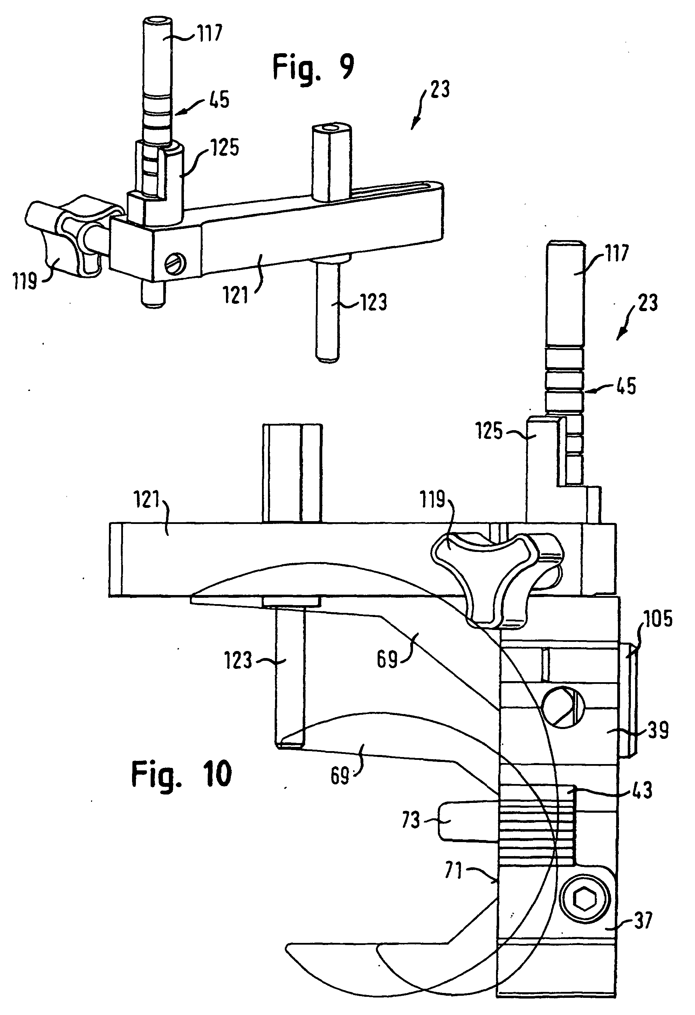

Der Femurtaster 23 gemäß Fig. 9 und 10 umfasst einen mit einer Skala 45 versehenen Stift 117, der auf das Oberteil 39 steckbar und dort mittels einer Feststellschraube 119 fixierbar ist. An einem sich senkrecht zum Stift 117 erstreckenden, gegenüber dem Stift 117 verdrehbaren Tastarm 121 ist ein Taststift 123 angebracht, der sich parallel zum Stift 117 und damit in Aufspreizrichtung erstreckt und in einem Langloch des Tastarms 121 längs des Tastarmes 121 verschiebbar ist. Bewegungen des Taststiftes 123 relativ zum Tastarm in Aufspreizrichtung sind nicht möglich.

Das in Fig. 9 und 10 untere freie Ende des Taststiftes 123 ruht während des Einsatzes der Flexionseinheit 19 auf der Vorderseite des in Flexion befindlichen Femurs. Relativbewegungen zwischen dem Oberteil 39 und dem Femur in Aufspreizrichtung, die bei freigegebenem Führungsstück 25 möglich sind, führen zu einer Verschiebung des Tastarmes 121 relativ zu dem Oberteil 39 und damit relativ zu dem auf das Oberteil 39 gesteckten Stift 117 und der an diesem angebrachten Skala 45. Ein Aufsatz 125 des Tastarmes 121 dient dabei als Zeiger, über den der jeweilige Skalenwert abgelesen werden kann.

Die Skala 45 ist derart gewählt, dass jeder Skalenstrich einer zur Verfügung stehenden Implantatgröße entspricht. Folglich kann bei freigegebenem Führungsstück 25 die Höhe des Oberteils 39 und damit des als Bohrlehre ausgebildeten Unterteils 37 relativ zum Femur derart eingestellt werden, dass diese Höhe exakt einer vorhandenen Implantatgröße entspricht, und zwar hinsichtlich der Größe des Implantats in der Richtung anterior-posterior. Diese Implantatgröße kann mit Hilfe der gestuften Flügelabschnitte 43 des Unterteils 37, an denen jede Stufe einer vorhandenen Implantatgröße entspricht, sowie mit Hilfe der am Träger 15 ausgebildeten Skala 47 (Fig. 1b) auf ihre Eignung als am besten passende Implantatgröße hin überprüft werden. Die Skala 47 wird also sowohl in Extension als auch in Flexion verwendet.

Um beim Verändern der Relativlage zwischen dem Oberteil 39 und dem Führungsstück 25 reproduzierbare Konfigurationen einstellen zu können, ist das Oberteil 39 mit einer Anzeigeeinrichtung 81 (Fig. 4b) in Form einer Skala versehen, an der z.B. über die untere Kante des Führungsstücks 25 ein Maß für dessen Höhe relativ zum Oberteil 39 ablesbar ist. Hierdurch steht dem Operateur folglich ein Maß für eine eventuell erforderliche Korrektureinstellung zur Verfügung, wenn sich beispielsweise bei in Flexion befindlichem Knie zeigt, dass eine zunächst in Erwägung gezogene Implantatgröße zu lockere Bänder zur Folge hätte, woraufhin die zunächst in Erwägung gezogene Schnitthöhe verändert wird, indem eine entsprechende Höhenverstellung des Oberteils 39 relativ zum Führungsstück 25 vorgenommen wird. Ein Maß für diese Änderung der Schnitthöhe ist dann an der Skala 81 ablesbar.

Die erfindungsgemäße Einstellvorrichtung ermöglicht die Auswahl einer am besten passenden Implantatgröße durch Auffinden der optimalen Position des als Bohrlehre ausgebildeten Unterteils 37 relativ zum Femur unter Berücksichtigung des korrekten Abstandes zwischen den Resektionsflächen der Tibia und des Femurs, unter Berücksichtigung der mediolateralen Ausrichtung des Unterteils 37 zum Femur sowie unter Berücksichtigung der vorhandenen Implantatgrößen, aus denen das für die jeweilige Operation am besten geeignete Implantat auszuwählen ist.

### Bezugszeichenliste

- 11: untere Arbeitseinheit
- 13: obere Arbeitseinheit
- 15: Träger
- 17: Extensionseinheit
- 19: Flexionseinheit
- 21: Basisabschnitt
- 23: Femurtaster
- 25: zentrales Führungstück
- 27: Führungskanal des Basisabschnitts
- 31: Arretierungsstrukturen des Führungsstücks
- 33: Arretierungseinrichtung des Basisabschnitts
- 35: Arretierungsbacke
- 37: Unterteil
- 39: Oberteil
- 41: Anzeigeeinrichtung des Unterteils
- 43: gestufter Flügelabschnitt des Unterteils
- 45: Anzeigeeinrichtung des Femurtasters
- 47: Anzeigeeinrichtung der unteren Arbeitseinheit
- 48: Zeiger des Trägers
- 49: Hebelmechanismus
- 51: Handgriff
- 52: Übergangsabschnitt
- 53: untere Abstützplatte
- 55: obere Abstützplatte
- 63: erster Hebel
- 65: zweiter Hebel
- 67: Feststelleinrichtung
- 68: Arretierungszahn
- 69: Femurimplantat
- 71: Innenfläche des Implantats
- 73: Verankerungsdorn des Implantats
- 75: Kopfteil
- 77: Tragstift
- 78: Tragstange
- 79: Verzahnung
- 81: Anzeigeeinrichtung des Basisabschnitts
- 83: Trägerdurchgang des Unterteils
- 85: Durchgang des Unterteils
- 87: Durchgang des Unterteils
- 89: Quersteg
- 91: Schlitzausnehmung
- 93: Querdurchgang des Oberteils
- 95: Querdurchgang des Unterteils
- 97: Spindel
- 99: Aufhängedurchgang des Führungsstücks
- 101: Sackbohrung
- 103: Querkanal
- 105: Dreh-/Druckschalter
- 106: Feder des Dreh-/Druckschalters
- 107: Verriegelungsstift
- 109: konische Steuerfläche
- 111: Feder der Arretierungsbacke
- 113: Stift der Arretierungsbacke
- 115: L-förmige Steuernut
- 117: Stift des Femurtasters
- 119: Feststellschraube
- 121: Tastarm
- 123: Taststift
- 125: Aufsatz

## Patentansprüche

1. Einstellvorrichtung zur Auswahl passender Implantatgrößen bei Knieoperationen mit
einer an der Tibia abstützbaren unteren Arbeitseinheit (11) und einer am Femur abstützbaren oberen Arbeitseinheit (13), die relativ zueinander höhenverstellbar sind und mit denen durch Aufspreizen des Knies der Abstand zwischen der Tibia und dem Femur einstellbar ist, **dadurch gekennzeichnet, daß**
die obere Arbeitseinheit (13) einen relativ zur unteren Arbeitseinheit (11) höhenverstellbaren Träger (15) und wenigstens zwei verschiedene Funktionseinheiten (17, 19) umfasst, die mit dem Träger (15) lösbar koppelbar sind und von denen die eine als zum Aufspreizen des Knies bei in Extension befindlichem Femur dienende Extensionseinheit (17) und die andere als zum Aufspreizen des Knies sowie zum Auswählen einer passenden Implantatgröße bei in Flexion befindlichem Femur dienende Flexionseinheit (19) ausgebildet ist,
wobei die Flexionseinheit (19) einen mit dem Träger (15) koppelbaren Basisabschnitt (21) und ein am Femur aufhängbares zentrales Führungsstück (25) umfasst, das wahlweise in einen relativ zum Basisabschnitt (21) in der Höhe frei bewegbaren oder festgesetzten Zustand bringbar ist, und
wobei bei festgesetztem Führungsstück (25) das Knie durch Verändern des Abstandes zwischen der unteren Arbeitseinheit (11) und dem Träger (15) aufspreizbar und bei frei bewegbarem Führungsstück (25) der Basisabschnitt (21) relativ zu dem am Femur aufhängbaren Führungsstück (25) in der Höhe verstellbar ist.

2. Einstellvorrichtung nach Anspruch 1,
**dadurch gekennzeichnet,**
**dass** das zentrale Führungsstück (25) um eine in Aufspreizrichtung verlaufende Achse relativ zum Basisabschnitt (21) verdrehbar ist.

3. Einstellvorrichtung nach Anspruch 2,
**dadurch gekennzeichnet,**
**dass** das zentrale Führungsstück (25) sowohl im frei bewegbaren als auch im festgesetzten Zustand relativ zum Basisabschnitt (21) verdrehbar ist.

4. Einstellvorrichtung nach einem der vorhergehenden Ansprüche,
**dadurch gekennzeichnet,**
**dass** das zentrale Führungsstück (25) in einem an zumindest zwei einander gegenüberliegenden Seiten offenen, in Aufspreizrichtung verlaufenden Führungskanal (27) des Basisabschnitts (21) in Längsrichtung des Führungskanals (27) verschiebbar und um eine Längsachse des Führungskanals (27) verdrehbar gelagert ist.

5. Einstellvorrichtung nach einem der vorhergehenden Ansprüche,
**dadurch gekennzeichnet,**
**dass** das zentrale Führungsstück (25) wenigstens einen Durchgang (99) aufweist, durch den eine Aufhängeeinrichtung, insbesondere ein Marknagel, zum Aufhängen des Führungsstücks (25) am in Flexion befindlichen Femur hindurch steckbar ist.

6. Einstellvorrichtung nach Anspruch 5,
**dadurch gekennzeichnet,**
**dass** der Durchgang (99) bezüglich einander diametral gegenüber liegender äußerer Funktionsbereiche, insbesondere Arretierungsstrukturen, schräg verläuft.

7. Einstellvorrichtung nach Anspruch 5 oder 6,
**dadurch gekennzeichnet,**
**dass** das zentrale Führungsstück (25) wenigstens zwei übereinander liegende Durchgänge (99) aufweist.

8. Einstellvorrichtung nach einem der vorhergehenden Ansprüche,
**dadurch gekennzeichnet,**
**dass** das zentrale Führungsstück (25) eine zylindrische Grundform aufweist.

9. Einstellvorrichtung nach einem der vorhergehenden Ansprüche,
**dadurch gekennzeichnet,**
**dass** die Außenseite des zentralen Führungsstücks (25) zumindest bereichsweise mit Arretierungsstrukturen (31) insbesondere in Form einer Verzahnung, Riffelung oder Nut-/Rippen-Anordnung versehen ist, mit denen eine Arretierungseinrichtung (33) des Basisabschnitts (21) zum wahlweisen Festsetzen oder Freigeben des Führungsstücks (25) in und außer Eingriff bringbar ist.

10. Einstellvorrichtung nach Anspruch 9,
**dadurch gekennzeichnet,**
**dass** die Arretierungseinrichtung (33) des Basisabschnitts (21) eine in und außer Eingriff mit dem zentralen Führungsstück (25) bewegbare Arretierungsbacke (35) umfasst, die derart auf die Arretierungsstrukturen (31) des Führungsstücks (25) abgestimmt ist, dass im Eingriffszustand eine Höhenverstellung des Führungsstück (25) relativ zum Basisabschnitt (21) unterbunden, ein Verdrehen des Führungsstücks (25) dagegen möglich ist.

11. Einstellvorrichtung nach Anspruch 9 oder 10,
**dadurch gekennzeichnet,**
**dass** die Arretierungseinrichtung (33) des Basisabschnitts (21) einen mit einem Finger betätigbaren kombinierten Dreh-/Druckschalter (105) umfasst.

12. Einstellvorrichtung nach einem der vorhergehenden Ansprüche,
**dadurch gekennzeichnet,**
**dass** der Basisabschnitt (21) ferner ein mit dem Träger (15) koppelbares Unterteil (37) und ein mit dem Unterteil (37) gekoppeltes Oberteil (39) umfasst, an dem das zentrale Führungsstück (25) gelagert und das zusammen mit dem zentralen Führungsstück (25) in mediolateraler Richtung relativ zum Unterteil (37) verstellbar ist.

13. Einstellvorrichtung nach Anspruch 12,
**dadurch gekennzeichnet,**
**dass** das Oberteil (39) als mit dem Unterteil (27) lösbar zusammengesteckter Schlitten ausgebildet ist, der am Unterteil (37) in mediolateraler Richtung verschiebbar gelagert ist.

14. Einstellvorrichtung nach Anspruch 12 oder 13,
**dadurch gekennzeichnet,**
**dass** das Unterteil (37) des Basisabschnitts (21) mit einer Anzeigeeinrichtung (41), insbesondere in Form zweier seitlicher gestufter Flügelabschnitte (43), versehen ist, an der das Femur durch Verstellen des Oberteils (39) in mediolateraler Richtung ausrichtbar ist, wobei vorzugsweise mittels der Anzeigeeinrichtung (41) bezüglich des ausgerichteten Femurs passende und nicht passende Implantatgrößen voneinander unterscheidbar sind.

15. Einstellvorrichtung nach einem der vorhergehenden Ansprüche,
**dadurch gekennzeichnet,**
**dass** die Flexionseinheit (19) ferner einen relativ zum Basisabschnitt (21) in der Höhe bewegbaren, zur Abstützung an der Vorderseite des in Flexion befindlichen Femurs ausgebildeten Femurtaster (23) umfasst.

16. Einstellvorrichtung nach Anspruch 15,
**dadurch gekennzeichnet,**
**dass** der Basisabschnitt (21) und/oder der Femurtaster (23) mit einer Anzeigeeinrichtung (45), insbesondere in Form einer Skala, versehen ist, an der ein von der Höhe des Femurtasters (23) relativ zu dem Basisabschnitt (21) abhängiges Maß für die Implantatgröße ablesbar ist.

17. Einstellvorrichtung nach einem der vorhergehenden Ansprüche,
**dadurch gekennzeichnet,**
**dass** der Träger (15) und/oder die untere Arbeitseinheit (11) mit einer Anzeigeeinrichtung (47), insbesondere in Form einer Skala, versehen ist, an der ein von der Höhe des Trägers (15) relativ zu der unteren Arbeitseinheit (11) abhängiges Maß für den Abstand zwischen der Tibia und dem Femur ablesbar ist.

18. Einstellvorrichtung nach einem der vorhergehenden Ansprüche,
**dadurch gekennzeichnet,**
**dass** der Basisabschnitt (21), insbesondere dessen Unterteil (37), als Bohr- und/oder Schneidlehre ausgebildet ist.

19. Einstellvorrichtung nach einem der vorhergehenden Ansprüche, **dadurch gekennzeichnet,**
**dass** die untere Arbeitseinheit (11) zur Höhenverstellung des Trägers (15) einen Hebelmechanismus (49) aufweist, der zusammen mit einem Handgriff (51) der unteren Arbeitseinheit (11) eine kombinierte Halte- und Betätigungseinrichtung bildet, die derart ausgelegt ist, dass ein Benutzer mit einer Hand die Einstellvorrichtung als Ganzes am Handgriff (51) halten und gleichzeitig den Hebelmechanismus (49) zur Höhenverstellung des Trägers (15) betätigen kann.

## Claims

1. An adjustment apparatus for the selection of suitable implant sizes in knee operations comprising
a lower work unit (11) supportable on the tibia and an upper work unit (13) supportable on the femur, which are vertically adjustable relative to one another and with which the spacing between the tibia and the femur can be adjusted by spreading apart the knee,
**characterized in that** the upper work unit (13) includes a carrier (15) vertically adjustable relative to the lower work unit (11) and at least two different functional units (17, 19) which are releasably couplable to the carrier (15) and of which the one is formed as an extension unit (17) serving for the spreading apart of the knee, when the femur is in extension, and the other is formed as a flexion unit (19) serving for the spreading apart of the knee and for the selection of a suitable implant size, when the femur is in flexion,
wherein the flexion unit (19) includes a base section (21) couplable to the carrier (15) and a central guide piece (25) which can be suspended at the femur and which can optionally be brought into a condition freely movable in height or fixed relative to the base section (21), and
wherein, when the guide piece (25) is fixed, the knee can be spread apart by changing the spacing between the lower work unit (11) and the carrier (15) and, when the guide piece (25) is freely movable, the base section (21) is adjustable in height relative to the guide piece (25) which can be suspended at the femur.

2. An adjustment apparatus in accordance with claim 1, **characterized in that** the central guide piece (25) is rotatable relative to the base section (21) about an axis extending in the spreading apart direction.

3. An adjustment apparatus in accordance with claim 2, **characterized in that** the central guide piece (25) is rotatable relative to the base section (21) both in the freely movable state and in the fixed state.

4. An adjustment apparatus in accordance with any one of the preceding claims, **characterized in that** the central guide piece (25) is supported in a guide passage (27) of the base section (21) open at at least two mutually opposite sides and extending in the direction of spreading apart, in a displaceable manner in the longitudinal direction of the guide passage (27) and in a rotatable manner about a longitudinal axis of the guide passage (27).

5. An adjustment apparatus in accordance with any one of the preceding claims, **characterized in that** the central guide piece (25) has at least one passage (99) through which a suspension device, in particular a medullary nail, can be inserted for the suspension of the guide piece (25) at the femur in flexion.

6. An adjustment apparatus in accordance with claim 5, **characterized in that** the passage (99) extends obliquely with respect to outer functional regions, in particular locking structures, lying diametrically opposite one another.

7. An adjustment apparatus in accordance with claim 5 or claim 6, **characterized in that** the central guide piece (25) has at least two passages (99) lying above one another.

8. An adjustment apparatus in accordance with any one of the preceding claims, **characterized in that** the central guide piece (25) has a cylindrical base shape.

9. An adjustment apparatus in accordance with any one of the preceding claims, **characterized in that** the external side of the central guide piece (25) is provided at least regionally with locking structures (31), in particular in the form of a toothed arrangement, a corrugation or a groove and rib arrangement, with which a locking device (33) of the base section (21) can be brought into and out of engagement for the alternative fixing or releasing of the guide piece (25).

10. An adjustment apparatus in accordance with claim 9, **characterized in that** the locking device (33) of the base section (21) includes a locking jaw (35) which can be brought into and out of engagement with the central guide piece (25) and which is matched to the locking structures (31) of the guide piece (25) such that a vertical adjustment of the guide piece (25) relative to the base section (21) is prevented in the engaged state, but a rotation of the guide piece (25) is possible.

11. An adjustment apparatus in accordance with claim 9 or claim 10, **characterized in that** the locking device (33) of the base section (21) includes a combined rotary/pressure switch (105) actuable with one finger.

12. An adjustment apparatus in accordance with any one of the preceding claims, **characterized in that** the base section (21) further includes a lower part (37) couplable to the carrier (15) and an upper part (39) which is coupled to the lower part (37), at which the central guide piece (25) is supported and which is adjustable in the mediolateral direction relative to the lower part (37) together with the central guide piece (25).

13. An adjustment apparatus in accordance with claim 12, **characterized in that** the upper part (39) is formed as a slide releasably plugged together with the lower part (27) and displaceably supported at the lower part (37) in the mediolateral direction.

14. An adjustment apparatus in accordance with claim 12 or claim 13, **characterized in that** the lower part (37) of the base section (21) is provided with a display device (41), in particular in the form of two lateral stepped wing sections (43), at which the femur can be aligned in the mediolateral direction by adjusting the upper part (39), with implant sizes suitable or not suitable with respect to the aligned femur preferably being able to be distinguished by means of the display device (41).

15. An adjustment apparatus in accordance with any one of the preceding claims, **characterized in that** the flexion unit (19) furthermore includes a femur probe (23) movable vertically relative to the base section (21) and made for support at the anterior side of the femur in flexion.

16. An adjustment apparatus in accordance with claim 15, **characterized in that** the base section (21) and/or the femur probe (23) is provided with a display device (45), in particular in the form of a scale, at which a measure for the implant size dependent on the height of the base section (21) relative to the femur probe (23) can be read off.

17. An adjustment apparatus in accordance with any one of the preceding claims, **characterized in that** the carrier (15) and/or the lower work unit (11) is/are provided with a display device (47), in particular in the form of a scale, at which a measure dependent on the height of the carrier (15) relative to the lower work unit (11) for the spacing between the tibia and the femur can be read off.

18. An adjustment apparatus in accordance with any one of the preceding claims, **characterized in that** the base section (21), in particular its lower part (37), is formed as a drill jig and/or as a cutting jig.

19. An adjustment apparatus in accordance with any one of the preceding claims, **characterized in that**, for the vertical adjustment of the carrier (15), the lower work unit (11) has a lever mechanism (49) which, together with a handle (51) of the lower work unit (11), forms a combined holding and actuating device which is made such that a user can hold the adjustment apparatus as a whole by the handle (51) and can simultaneously actuate the lever mechanism (49) for the vertical adjustment of the carrier (15).

## Revendications

1. Dispositif de réglage pour le choix de tailles d'implants appropriées pour les opérations du genou, comprenant
une unité de travail inférieure (11) pouvant prendre appui sur le tibia et une unité de travail supérieure (13) pouvant prendre appui sur le fémur, celles-ci pouvant se déplacer en hauteur l'une par rapport à l'autre et permettant d'ajuster la distance entre le tibia et le fémur par déploiement du genou,
**caractérisé en ce que**
l'unité de travail supérieure (13) comprend un support (15) pouvant se déplacer en hauteur par rapport à l'unité de travail inférieure (11), et au moins deux unités fonctionnelles différentes (17, 19) pouvant former un couplage amovible avec le support (15), l'une d'elles constituant une unité d'extension (17) destinée au déploiement du genou lorsque le fémur se trouve en extension, l'autre constituant une unité de flexion (19) destinée au déploiement du genou et à la sélection d'une taille d'implant appropriée lorsque le fémur est en flexion, l'unité de flexion (19) comprenant une section de base (21) pouvant être couplée au support (15), ainsi qu'un élément de guidage central (25) pouvant être accroché au fémur et pouvant être placé au choix de façon à pouvoir se déplacer librement en hauteur ou dans une position fixe par rapport à la section de base (21), et lorsque l'élément de guidage (25) est en position fixe, le genou peut être déployé par modification de la distance entre l'unité de travail inférieure (11) et le support (15) et, lorsque l'élément de guidage (25) peut se déplacer librement, la section de base (21) peut être ajustée en hauteur par rapport à l'élément de guidage (25) accroché au fémur.

2. Dispositif de réglage selon la revendication 1,
**caractérisé en ce que**
l'élément de guidage central (25) peut pivoter par rapport à la section de base (21) autour d'un axe orienté dans le sens du déploiement.

3. Dispositif de réglage selon la revendication 2,
**caractérisé en ce que**
l'élément de guidage central (25), qu'il soit en position fixe ou mobile, peut pivoter par rapport à la section de base (21).

4. Dispositif de réglage selon l'une des revendications précédentes,
**caractérisé en ce que**
l'élément de guidage central (25) est disposé de manière à se déplacer à l'intérieur d'un canal de guidage (27) de la section de base (21) s'étendant dans le sens du déploiement, dans le sens longitudinal du canal de guidage, lequel est ouvert sur au moins deux extrémités opposées, et à pouvoir pivoter autour d'un axe longitudinal du canal de guidage (27).

5. Dispositif de réglage selon l'une des revendications précédentes,
**caractérisé en ce que**
l'élément de guidage central (25) présente au moins une ouverture (99) dans laquelle il est possible d'introduire de part en part un système d'attache, en particulier une broche, destiné à accrocher l'élément de guidage (25) sur le fémur qui se trouve en flexion.

6. Dispositif de réglage selon la revendication 5,
**caractérisé en ce que**
l'ouverture (99) est en position oblique par rapport à des zones fonctionnelles externes, en particulier des structures d'arrêt, en position diamétralement opposées l'une par rapport à l'autre.

7. Dispositif de réglage selon la revendication 5 ou 6,
**caractérisé en ce que**
l'élément de guidage central (25) présente au moins deux ouvertures (99) l'une au-dessus de l'autre.

8. Dispositif de réglage selon l'une des revendications précédentes,
**caractérisé en ce que**
l'élément de guidage central (25) présente une forme de base cylindrique.

9. Dispositif de réglage selon l'une des revendications précédentes,
**caractérisé en ce que**
la partie externe de l'élément de guidage central (25) est pourvue, dans certaines zones tout au moins, de structures d'arrêt (31), en particulier sous la forme d'une denture, de stries ou d'une succession d'encoches et de nervures, permettant l'engagement et le dégagement d'un dispositif d'arrêt (33) de la section de base (21) pour immobiliser ou libérer au choix l'élément de guidage (25).

10. Dispositif de réglage selon la revendication 9,
**caractérisé en ce que**
le dispositif d'arrêt (33) de la section de base (21) comprend une joue d'arrêt (35) pouvant se déplacer avec l'élément de guidage central (25) en position engagée ou dégagée, celle-ci étant ajustée en fonction des structures d'arrêt (31) de l'élément de guidage (25) de façon à interdire, en position engagée, un déplacement en hauteur de l'élément de guidage (25) par rapport à la section de base (21) tout en permettant une rotation de l'élément de guidage (25).

11. Dispositif de réglage selon la revendication 9 ou 10,
**caractérisé en ce que**
le dispositif d'arrêt (33) de la section de base (21) comprend un bouton rotatif / à poussoir combiné (105), pouvant être actionné avec un doigt.

12. Dispositif de réglage selon l'une des revendications précédentes,
**caractérisé en ce que**
la section de base (21) comprend en outre une partie inférieure (37) pouvant être couplée au support (15) et une partie supérieure (39) pouvant être couplée à la partie inférieure (37), sur laquelle l'élément de guidage central (25) est disposé, et pouvant être ajustée avec l'élément de guidage central (25) dans un sens médio-latéral par rapport à la partie inférieure (37).

13. Dispositif de réglage selon la revendication 12,
**caractérisé en ce que**
la partie supérieure (39) constitue un chariot présentant un assemblage amovible avec la partie inférieure (27), ce chariot étant disposé de façon à pouvoir se déplacer dans un sens médio-latéral sur la pièce inférieure (37).

14. Dispositif de réglage selon la revendication 12 ou 13,
**caractérisé en ce que**
la partie inférieure (37) de la section de base (21) est pourvue d'un dispositif de visualisation (41), présentant en particulier la forme de deux sections d'aile (43) latéralement étagées, sur lequel le fémur peut être centré dans un sens médio-latéral par déplacement de la partie supérieure (39), pour permettre, de préférence à l'aide d'un dispositif de visualisation (41), de distinguer les tailles d'implant appropriées et inappropriées par rapport au fémur centré.

15. Dispositif de réglage selon l'une des revendications précédentes,
**caractérisé en ce que**
l'unité de flexion (19) comprend par ailleurs un palpeur fémoral (23) pouvant se déplacer en hauteur par rapport à la section de base (21), et s'appuyant contre la partie frontale du fémur en flexion.

16. Dispositif de réglage selon la revendication 15,
**caractérisé en ce que**
la section de base (21) et/ou le palpeur fémoral (23) sont pourvus d'un dispositif de visualisation (45), en particulier sous la forme d'une graduation, sur lequel il est possible de relever une mesure pour la taille de l'implant, qui varie en fonction de la hauteur du palpeur fémoral (23) par rapport à la section de base (21).

17. Dispositif de réglage selon l'une des revendications précédentes,
**caractérisé en ce que**
le support (15) et/ou l'unité de travail inférieure (11) sont pourvus d'un dispositif de visualisation (47), en particulier sous la forme d'une graduation, sur lequel il est possible de relever une mesure pour la distance entre le tibia et le fémur, qui varie en fonction de la hauteur du support (15) par rapport à l'unité de travail inférieure (11).

18. Dispositif de réglage selon l'une des revendications précédentes,
**caractérisé en ce que**
la section de base (21), en particulier sa partie inférieure (37), constitue un gabarit de perçage et/ou de coupage.

19. Dispositif de réglage selon l'une des revendications précédentes,
**caractérisé en ce que**
l'unité de travail inférieure (11) pour l'ajustement en hauteur du support (15) présente un mécanisme de levier (49) qui, en liaison avec une poignée (51) de l'unité de travail inférieure (11) forme un dispositif d'actionnement et d'arrêt combiné, conçu de telle façon qu'un utilisateur puisse tenir d'une main l'ensemble du dispositif de réglage par la poignée (15) et, simultanément, actionner le mécanisme de levier (49) pour l'ajustement en hauteur du support (15).
